(19) Europäisches Patentamt
European Patent Office
Office européen des brevets

(11) EP 4 279 485 A1

(12) EUROPEAN PATENT APPLICATION
published in accordance with Art. 153(4) EPC

(43) Date of publication:
**22.11.2023 Bulletin 2023/47**

(21) Application number: **22739019.2**

(22) Date of filing: **12.01.2022**

(51) International Patent Classification (IPC):
***C07D 239/42*** (2006.01) ***C07D 403/12*** (2006.01)
***A61K 31/506*** (2006.01) ***A61P 37/00*** (2006.01)

(52) Cooperative Patent Classification (CPC):
**A61K 31/506; A61P 37/00; C07D 239/42; C07D 403/12**

(86) International application number:
**PCT/CN2022/071491**

(87) International publication number:
**WO 2022/152140 (21.07.2022 Gazette 2022/29)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **14.01.2021 CN 202110048366**

(71) Applicant: **The National Institutes of Pharmaceutical R&D Co., Ltd**
**Changping District**
**Beijing 102206 (CN)**

(72) Inventors:
- **YIN, Huijun**
  **Beijing 102206 (CN)**
- **YAN, Xu**
  **Beijing 102206 (CN)**
- **SHI, Jizhou**
  **Beijing 102206 (CN)**
- **LIU, Guobiao**
  **Beijing 102206 (CN)**
- **DONG, Liuxin**
  **Beijing 102206 (CN)**
- **SHAO, Mingzhao**
  **Beijing 102206 (CN)**

(74) Representative: **EP&C**
**P.O. Box 3241**
**2280 GE Rijswijk (NL)**

# (54) BRIDGED HETEROCYCLYL-SUBSTITUTED PYRIMIDINE COMPOUNDS, PREPARATION METHOD AND MEDICAL USE THEREOF

(57) Bridged heterocyclyl-substituted pyrimidine compounds, a preparation method and medical use thereof. The compounds or pharmaceutical compositions comprising same can be used as JAK1 and TYK2 kinase inhibitors for treating JAK1 and TYK2 kinase activity-related diseases such as inflammations, autoimmune diseases and cancers.

EP 4 279 485 A1

## Description

### FIELD OF THE INVENTION

The present invention belongs to the medical technical field, and specifically relates to a bridged heterocyclyl-substituted pyrimidine compound, a method for preparing the same and a pharmaceutical composition containing the same, as well as use thereof for regulating Janus kinase 1 (JAK1) and tyrosine protein kinase 2 (TYK2) activity and use thereof in treating and/or preventing diseases related to JAK1 and TYK2 activity.

### BACKGROUND OF THE INVENTION

The process of intracellular signaling is an effective way for cells to respond to external stimulus and ultimately trigger specific biological effects. Cytokines can carry out intracellular signaling through a variety of signaling pathways, thereby being involved in the regulation of hematopoietic function and many important biological functions related to immunity. The Janus kinase (JAK) family of protein tyrosine kinases and transcriptional activators (STAT) play an important role in the process of cytokine signaling (J. Immunol. 2015, 194, 21).

The Janus kinase (JAK) family plays a certain role in the cytokine-dependent regulation of cell proliferation and function involved in immune response. Currently, there are four known mammalian JAK family members: JAK1 (also known as Janus kinase-1), JAK2 (also known as Janus kinase-2), JAK3 (also known as Janus kinase, leukocyte, JAKL1, L-JAK and Janus kinase-3), Tyk2 (also known as protein-tyrosine kinase 2). JAK1, JAK2 and Tyk2 are widely present in various tissues and cells, while JAK3 is only present in the bone marrow and lymphatic system (J. Med. Chem. 2014, 57, 5023).

Tyk2 is the first J discovered AK kinase. It plays an important role in regulating the biological response of IL-12 and bacterial lipopolysaccharide (LPS), and is also involved in signaling pathways mediated by IL-6, IL-10 and IL-12. Targeting Tyk2 can become a new strategy for treating diseases mediated by IL-12, IL-23 or type I IFN. Said diseases include but are not limited to rheumatoid arthritis, multiple sclerosis, lupus, psoriasis, psoriatic arthritis, inflammatory bowel disease, uveitis, sarcoidosis, lupus erythematosus and cancer.

JAK1 plays an important role in regulating the biological response function of multiple cytokine receptor families. JAK1 gene knockout mice have an early postnatal lethal factor phenotype, and the nervous system is also damaged, resulting in birth defects in young mice. Studies have shown that JAK1 gene knockout mice will have thymocyte and B cell secretion defects, and JAK1 gene knockout tissues have significantly weakened response to LIF, IL-6 and IL-10. Clinical trials have shown that JAK1 inhibitors have shown good efficacy in treating inflammatory and autoimmune diseases such as rheumatoid arthritis, ulcerative colitis, Crohn’s disease, lupus erythematosus, alopecia areata, atopic dermatitis.

Upon cytokine binding to receptor, the receptor forms a dimer which approaches the JAK coupled with the receptor to active JAK by phosphorylation of tyrosine residues. In turn, it catalyzes the phosphorylation of the tyrosine residues of the receptor itself, forming a "docking site". Signal Transducer and Activator of Transcription (STAT) is a group of cytoplasmic proteins that can regulate target genes and bind with DNA. The STAT family includes STAT1, STAT2, STAT3, STAT4, STAT5a, STAT5b and StAT6. STAT recognizes the "docking site" through the SH2 domain, and is activated by phosphorylation of its C-terminus tyrosine residue by JAK kinase. The activated STAT factor is transferred into the nucleus and plays an important role in regulating the innate and acquired host immune response.

The activation of JAK/STAT signaling pathway promotes the occurrence of various diseases, including but not limited to, many abnormal immune responses, such as allergies, asthma, rheumatoid arthritis, amyotrophic lateral sclerosis, multiple sclerosis and the like. It is also associated with cancers such as leukemia (acute myeloid leukemia and acute lymphoblastic leukemia) and solid tumors (uterine leiomyosarcoma, prostate cancer) (Curr. Opin. Rheumatol. 2014, 26, 237).

In view of the important roles that JAK1 and TYK2 play in the inflammatory signaling pathways, drugs that can simultaneously inhibit both kinases have the potential of further enhancing the efficacy and bringing greater benefits to patients.

### SUMMARY OF THE INVENTION

Through intensive research, the inventors have designed and synthesized a series of bridged heterocyclyl-substituted pyrimidine compounds, which have been screened for JAK1 and TYK2 activities. The research results show that these compounds have outstanding JAK1 and TYK2 inhibitory activities, and can be developed as a medicament for treating diseases related to JAK1 and TYK2 activity.

The objective of the present invention is to provide a compound or a pharmaceutically acceptable salt thereof selected from the group consisting of:

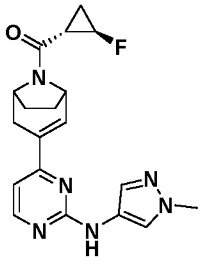, 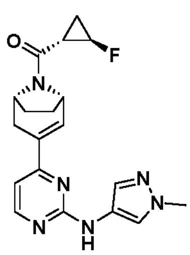, 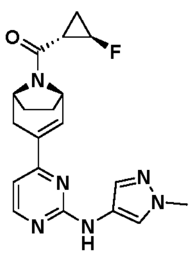, 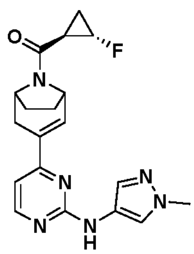, 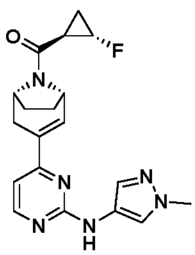

and

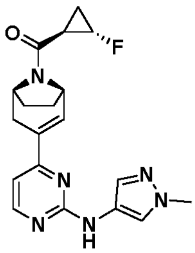.

The present invention further provides a method for preparing the compound of formula 1 or a pharmaceutically acceptable salt thereof, comprising the following step of:

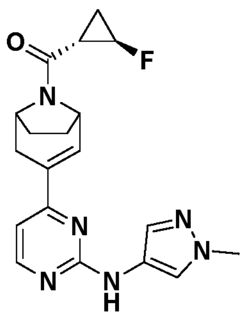

1

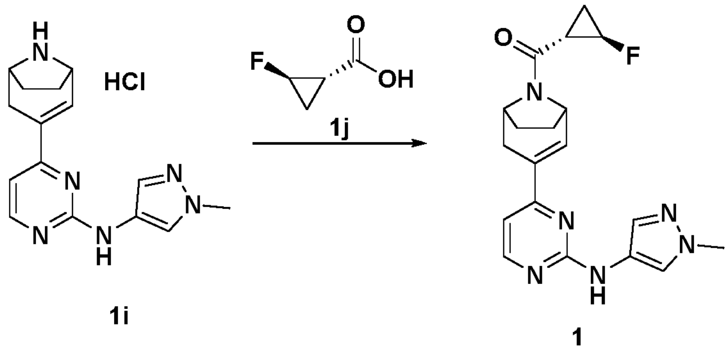


1i

1

reacting the hydrochloride of compound **1i** with compound 1j under alkaline conditions and the presence of a catalyst to obtain compound 1; the reagent providing alkaline conditions is preferably DIEA, the catalyst is preferably HATU.

The present invention also provides a method for preparing the compound of formula 2 or a pharmaceutically acceptable salt thereof, comprising the following step of:

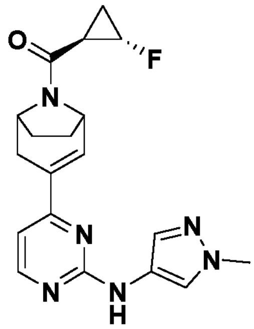

**2**

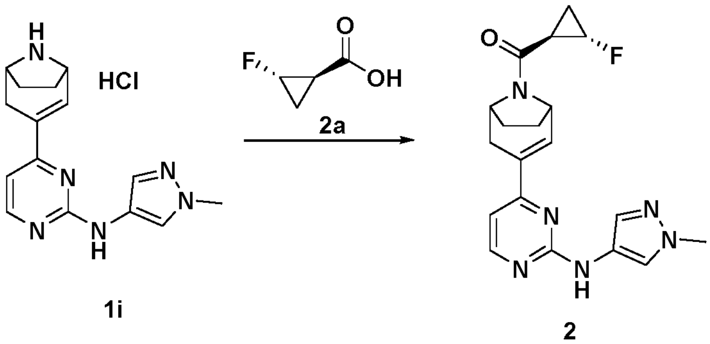


**1i** **2**

reacting compound **1i** with compound **2** under alkaline conditions and the presence of a catalyst to obtain compound **2;** the reagent providing alkaline conditions is preferably DIEA, the catalyst is preferably HATU.

The present invention also provides a method for preparing the compound of formula 1-a and/or 1-b or a pharmaceutically acceptable salt thereof, comprising the following step of:

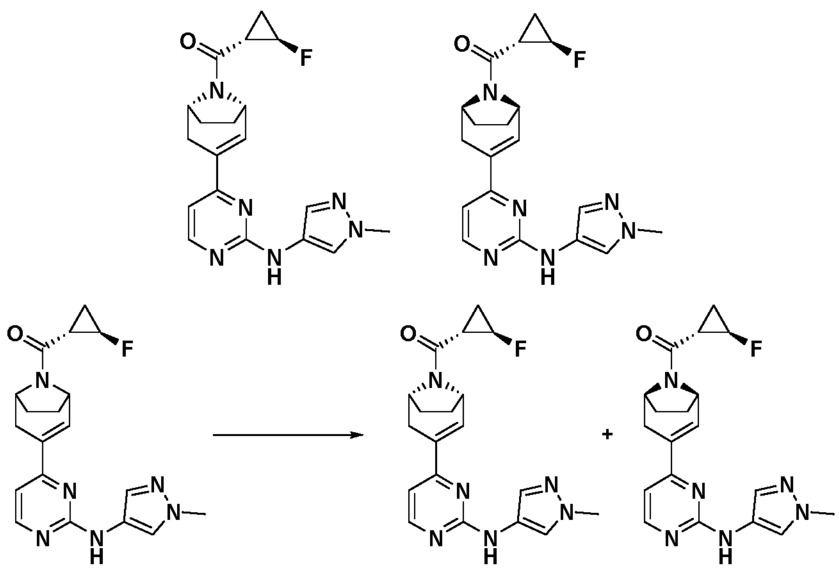


**1**

resolving compound **1** by supercritical fluid chromatography to obtain compound 1-a and/or compound 1-b; wherein the one with shorter retention time is compound 1-a, and the one with longer retention time is compound 1-b.

The present invention further provides a method for preparing the compound of formula 2-a and/or 2-b or a pharmaceutically acceptable salt thereof, comprising the following step of:

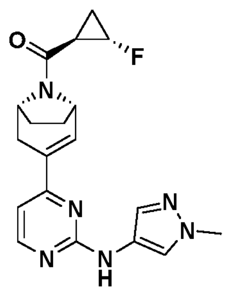

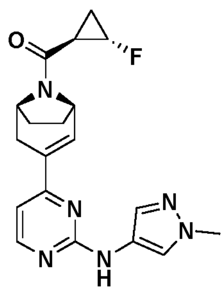

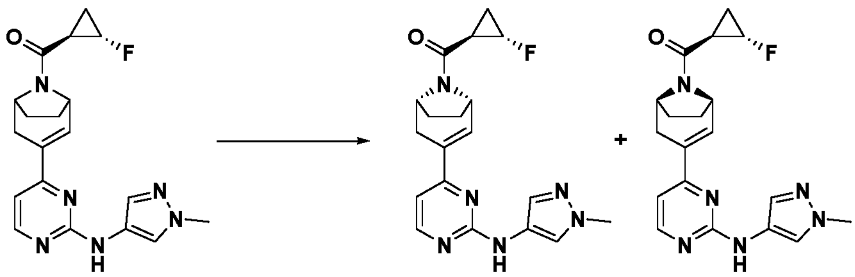

2

resolving compound 2 by supercritical fluid chromatography to obtain compound 2-a and/or compound 2-b; wherein the one with shorter retention time is compound 2-a, and the one with longer retention time is compound 2-b.

The supercritical fluid chromatography of the present invention is a chromatography with supercritical fluid as mobile phase. Supercritical fluid refers to substances that are neither gas nor liquid, and their physical properties are between gas and liquid.

Supercritical fluid chromatography of the present invention preferably uses methanol and carbon dioxide as mobile phase, more preferably uses methanol containing ammonia and carbon dioxide as mobile phase, particularly uses MeOH (0.2% $NH_3.H_2O$)/$CO_2$ as mobile phase, and more particularly uses MeOH (0.2% $NH_3 \cdot H_2O$)/$CO_2$ at a volume ratio of 40:60 as mobile phase.

The present invention further provides a pharmaceutical composition comprising the compound of the present invention and a pharmaceutically acceptable carrier.

The present invention further relates to a use of the compound or the pharmaceutical composition comprising the same according to the present invention in the preparation of JAK1 and TYK2 inhibitors.

The present invention further relates to a use of the compound or a pharmaceutically acceptable salt thereof or the pharmaceutical composition comprising the same according to the present invention in the preparation of medicaments for the prevention and/or treatment of diseases related to JAK1 and TYK2 activity, wherein the disease is selected from the group consisting of inflammation, autoimmune disease and cancer, and the inflammation is preferably selected from the group consisting of rheumatoid arthritis, psoriatic arthritis, inflammatory bowel disease, uveitis, psoriasis and atopic dermatitis, the autoimmune disease is preferably selected from the group consisting of multiple sclerosis and lupus; the cancer is preferably selected from the group consisting of breast cancer, cervical cancer, colon cancer, lung cancer, gastric cancer, rectal cancer, pancreatic cancer, brain cancer, skin cancer, oral cancer, prostate cancer, bone cancer, kidney cancer, ovarian cancer, bladder cancer, liver cancer, fallopian tube tumor, ovarian tumor, peritoneal tumor, melanoma, solid tumor, glioma, glioblastoma, hepatocellular carcinoma, mastoid nephroma, head and neck tumors, leukemia, lymphoma, myeloma and non-small cell lung cancer.

The present invention further relates to the compound or a pharmaceutically acceptable salt thereof or the pharmaceutical composition comprising the same according to the present invention, for use as a drug.

The present invention further relates to the compound or a pharmaceutically acceptable salt thereof or the pharmaceutical composition comprising the same according to the present invention, for use as a JAK1 and TYK2 inhibitor.

The present invention further relates to the compound or a pharmaceutically acceptable salt thereof or the pharmaceutical composition comprising the same according to the present invention, for use in the prevention and/or treatment of disease related to JAK1 and TYK2 activity, wherein the disease is selected from the group consisting of inflammation, autoimmune disease and cancer, and the inflammation is preferably selected from the group consisting of rheumatoid arthritis, psoriatic arthritis, inflammatory bowel disease, uveitis, psoriasis and atopic dermatitis, the autoimmune disease is preferably selected from the group consisting of multiple sclerosis and lupus; the cancer is preferably

selected from the group consisting of breast cancer, cervical cancer, colon cancer, lung cancer, gastric cancer, rectal cancer, pancreatic cancer, brain cancer, skin cancer, oral cancer, prostate cancer, bone cancer, kidney cancer, ovarian cancer, bladder cancer, liver cancer, fallopian tube tumor, ovarian tumor, peritoneal tumor, melanoma, solid tumor, glioma, glioblastoma, hepatocellular carcinoma, mastoid nephroma, head and neck tumors, leukemia, lymphoma, myeloma and non-small cell lung cancer.

The present invention further relates to a method for inhibiting JAK1 and TYK2, comprising contacting the compound or a pharmaceutically acceptable salt thereof or the pharmaceutical composition comprising the same according to the present invention with JAK1 and TYK2.

The present invention further relates to a method for preventing and/or treating diseases related to JAK1 and TYK2 activity, comprising administering a therapeutically effective amount of the compound or a pharmaceutically acceptable salt thereof or the pharmaceutical composition comprising the same according to the present invention to a subject in need thereof, wherein the disease is selected from the group consisting of inflammation, autoimmune disease and cancer, and the inflammation is preferably selected from the group consisting of rheumatoid arthritis, psoriatic arthritis, inflammatory bowel disease, uveitis, psoriasis and atopic dermatitis, the autoimmune disease is preferably selected from the group consisting of multiple sclerosis and lupus; the cancer is preferably selected from the group consisting of breast cancer, cervical cancer, colon cancer, lung cancer, gastric cancer, rectal cancer, pancreatic cancer, brain cancer, skin cancer, oral cancer, prostate cancer, bone cancer, kidney cancer, ovarian cancer, bladder cancer, liver cancer, fallopian tube tumor, ovarian tumor, peritoneal tumor, melanoma, solid tumor, glioma, glioblastoma, hepatocellular carcinoma, mastoid nephroma, head and neck tumors, leukemia, lymphoma, myeloma and non-small cell lung cancer.

According to the conventional methods in the field of the present invention, the compound of the present invention can be formed in a pharmaceutically acceptable acid addition salt with an acid. The acid includes inorganic acids and organic acids, particularly preferably hydrochloric acid, hydrobromic acid, sulfuric acid, phosphoric acid, methanesulfonic acid, ethanesulfonic acid, p-toluenesulfonic acid, benzenesulfonic acid, naphthalene disulfonic acid, acetic acid, propionic acid, lactic acid, trifluoroacetic acid, maleic acid, citric acid, fumaric acid, oxalic acid, tartaric acid, benzoic acid and the like.

According to conventional methods in the field of the present invention, the compound of the present invention can be formed in a pharmaceutically acceptable basic addition salt with a base. The base includes inorganic bases and organic bases. Acceptable organic bases include diethanolamine, ethanolamine, N-methylglucamine, triethanolamine, tromethamine and the like, and acceptable inorganic bases include aluminum hydroxide, calcium hydroxide, potassium hydroxide, sodium carbonate, sodium hydroxide and the like.

The pharmaceutical composition comprising the active ingredient can be in a form suitable for oral administration, for example a tablet, troche, lozenge, aqueous or oily suspension, dispersible powder or granule, emulsion, hard or soft capsule, syrup or elixir. An oral composition can be prepared according to any method known in the art for preparing a pharmaceutical composition, and such compositions can also comprise one or more components selected from the group consisting of sweetener, flavoring agent, coloring agent and preservative, in order to provide a pleasing and palatable pharmaceutical preparation. The tablet contains the active ingredient in admixture with non-toxic pharmaceutically acceptable excipients suitable for the manufacture of tablets. These excipients can be inert excipients, such as calcium carbonate, sodium carbonate, lactose, calcium phosphate or sodium phosphate; granulating and disintegrating agents, for example microcrystalline cellulose, croscarmellose sodium, corn starch or alginic acid; binders, for example starch, gelatin, polyvinylpyrrolidone or arabic gum; and lubricants, for example magnesium stearate, stearic acid or talc. The tablet can be uncoated or coated by a known technique to mask the taste of the drug or delay the disintegration and absorption of the active ingredient in the gastrointestinal tract, thereby providing a sustained release over a long period of time. For example, water-soluble taste-masking substances such as hydroxypropyl methylcellulose or hydroxypropyl cellulose, or time-extending substances such as ethylcellulose or cellulose acetate butyrate can be used.

An oral formulation can also be provided as a hard gelatin capsules in which the active ingredient is mixed with an inert solid diluent such as calcium carbonate, calcium phosphate or kaolin, or a soft gelatin capsules in which the active ingredient is mixed with a water-soluble carrier such as polyethylene glycol, or an oil solvent such as peanut oil, liquid paraffin or olive oil.

An aqueous suspension comprises an active ingredient in admixture with excipients suitable for the manufacture of an aqueous suspension. Such excipients are suspending agents such as sodium carboxymethylcellulose, methylcellulose, hydroxypropylmethylcellulose, sodium alginate, polyvinylpyrrolidone and arabic gum; dispersing or wetting agents, which may be a naturally occurring phospholipid, such as lecithin, or a condensation product of alkylene oxide with fatty acid, such as polyoxyethylene stearate, or a condensation product of ethylene oxide with long chain fatty alcohol, such as heptadecylethyleneoxy cetanol, or a condensation product of ethylene oxide with partial ester derived from fatty acid and hexitol, such as polyethylene oxide sorbitol monooleate, or a condensation product of ethylene oxide with partial ester derived from fatty acid and hexitol anhydride, such as polyethylene oxide sorbitan monooleate. The aqueous suspension can also comprise one or more preservatives, such as ethylparaben or n-propylparaben, one or

more coloring agents, one or more flavoring agents and one or more sweetening agents, such as sucrose, saccharin or aspartame.

An oil suspension can be formulated by suspending the active ingredient in a vegetable oil such as peanut oil, olive oil, sesame oil or coconut oil, or a mineral oil, such as liquid paraffin. The oil suspension can comprise a thickener, such as beeswax, hard paraffin or cetyl alcohol. The aforementioned sweeteners and flavoring agents can be added to provide a palatable preparation. The compositions can be kept by adding an antioxidant, such as butylated hydroxyanisole or alpha-tocopherol.

The dispersible powders or granules suitable for the preparation of an aqueous suspension can provide the active ingredient in admixture with the dispersants or wetting agents, suspending agent or one or more preservatives by adding water. Suitable dispersants or wetting agents and suspending agents are as described above. Additional excipients, such as sweeteners, flavoring agents and colorants can also be added. The compositions can be kept by adding an antioxidant, such as ascorbic acid.

The pharmaceutical composition of the present invention can also be in the form of an oil-in-water emulsion. The oil phase can be a vegetable oil, such as olive oil or peanut oil, or a mineral oil, such as liquid paraffin, or a mixture thereof. Suitable emulsifiers can be naturally occurring phospholipids, such as soy lecithin, and esters or partial esters derived from fatty acids and hexitol anhydrides, such as sorbitan monooleate, and condensation products of the partial ester and ethylene oxide, such as polyethylene oxide sorbitol monooleate. The emulsion can also comprise sweeteners, flavoring agents, preservatives and antioxidants. Acceptable sweeteners are for example syrups and elixirs prepared with glycerol, propylene glycol, sorbitol or sucrose. Such preparations can also comprise demulcents, preservatives, colorants and antioxidants.

The pharmaceutical composition of the present invention can also be in the form of a sterile injectable aqueous solution. Acceptable vehicles or solvents that can be used are water, Ringer's solution or isotonic sodium chloride solution. The sterile injectable preparation can be a sterile injectable oil-in-water microemulsion in which the active ingredient is dissolved in the oil phase. For example, the active ingredient is dissolved in a mixture of soybean oil and lecithin, which is then added to a mixture of water and glycerol to form a microemulsion. The injection solution or microemulsion can be introduced into the bloodstream of patients by local bolus injection. Alternatively, the solution and micro-emulsion are preferably administered in a manner that maintains a constant circulating concentration of the compound of the present invention. In order to maintain this constant concentration, a continuous intravenous delivery device can be used.

The pharmaceutical composition of the present invention can be in the form of a sterile injectable aqueous or oil suspension for intramuscular and subcutaneous administration. Such a suspension can be formulated with suitable dispersants or wetting agents and suspending agents as described above according to known techniques. The sterile injectable formulation can also be a sterile injectable solution or suspension prepared in a non-toxic parenterally acceptable diluent or solvent, for example a solution prepared in 1,3-butanediol. In addition, a sterile fixed oil can be conveniently used as a solvent or suspension medium. For this purpose, any blended fixed oil including synthetic mono- or diglycerides can be used. In addition, fatty acids, for example oleic acid, can also used to prepare the injections.

The compound of the present invention can be administered in the form of a suppository for rectal administration. These pharmaceutical compositions can be prepared by mixing the drug with a suitable non-irritating excipient, which is solid at ordinary temperature but is liquid in the rectum, thereby melting in the rectum to release the drug. Such substances include cocoa butter, glycerin gelatin, hydrogenated vegetable oil, and a mixture of polyethylene glycol and fatty acid esters of polyethylene glycol of various molecular weights.

It is well known to those skilled in the art that the dosage of a drug depends on a variety of factors including, but not limited to the following factors: the activity of the specific compound, the age, weight, health condition, behavior and diet of the patient, administration time, administration route, excretion rate, drug combination and the like. Moreover, the optimal treatment, such as treatment mode, daily dose of the compound or the type of the pharmaceutically acceptable salt, can be verified according to the conventional therapeutic regimens.

The present invention can comprise a composition prepared with the compound, or a pharmaceutically acceptable salt, hydrate or solvate thereof as an active ingredient in admixture with a pharmaceutically acceptable carrier or excipient, which can be formulated into a clinically acceptable formulation.

The derivatives of the present invention can be used in combination with other active ingredients, as long as they do not exert adverse effects, for example allergic reactions and the like. The compound of the present invention can be used as the only active ingredient, or can also be used in combination with other active ingredient for the treatment of diseases related to JAK1 and TYK2 activity. Combination therapy is achieved by administering each active ingredients simultaneously, separately or sequentially.

In the present disclosure, "pharmaceutical composition" refers to a mixture containing one or more of the compounds described herein, or a physiologically/pharmaceutically acceptable salt or a prodrug thereof, and other chemical components, as well as other components, such as physiological/pharmaceutically acceptable carrier and excipient. The purpose of the pharmaceutical composition is to facilitate administration of a compound to an organism,

which is conducive to the absorption of the active ingredient so as to show the biological activity.

In the present disclosure, "pharmaceutically acceptable salt" refers to a salt of the compound of the present invention, which is safe and effective for use in mammals and possesses the desired biological activity.

## DESCRIPTION OF THE DRAWING

Figure 1 is a graph showing the rat AIA pharmacodynamic scoring results of the compounds of the present invention.

## DETAILED DESCRIPTION OF THE INVENTION

The present invention will be further described with reference to the following examples, but the examples should not be considered as limiting the scope of the present invention.

The structures of the compounds are identified by nuclear magnetic resonance (NMR) and/or mass spectrometry (MS). NMR shift is given in $10^{-6}$ (ppm). NMR is determined by a Brukerdps300 nuclear magnetic spectrometer. The solvents for determination are deuterated dimethyl sulfoxide (DMSO-d6), deuterated chloroform ($CDCl_3$), deuterated methanol ($CD_3OD$), and the internal standard is tetramethylsilane (TMS).

MS is determined by a 1100 Series LC/MSD Trap (ESI) mass spectrometer (manufacturer: Agilent).

Unless specified in the examples, a 1c3000 high performance liquid chromatograph and a 1c6000 high performance liquid chromatograph (manufacturer: ChuangXin TongHeng) are used for preparative liquid chromatograph. The chromatographic column is DaisogelC18 10 μm 60 A (20 mm×250 mm). Mobile phase: acetonitrile, water (0.05% formic acid).

HPLC is determined by a Shimadzu LC-20AD high pressure liquid chromatograph (Agilent TC-C18 250×4.6 mm 5 μm column) and a Shimadzu LC-2010AHT high pressure liquid chromatograph (Phenomenex C18 250×4.6 mm 5 μm column).

Qingdao Haiyang Chemical GF254 silica gel plates are used for thin layer chromatography (TLC), and the specification is 0.15 mm to 0.2 mm for analysis, and 0.4 mm to 0.5 mm for separation and purification of products.

Qingdao Haiyang 100 to 200 mesh and 200 to 300 mesh silica gel are generally used as the carrier for column chromatography.

The known starting materials of the present invention can be synthesized by or according to methods known in the art, or can be purchased from WHall, Beijing Ouhe, Sigma, J&K Scientific, Yishiming, Shanghai Shuya, Innochem, Nanjing Pharmablock, Energy Chemical and other companies.

Unless specified in the examples, all reactions can be carried out under argon atmosphere or nitrogen atmosphere.

Argon atmosphere or nitrogen atmosphere means that the reaction flask is connected to an argon or nitrogen balloon with a volume of about 1 L.

A CEM Discover SP microwave reactor is used for microwave reaction.

Unless specified in the examples, a solution refers to an aqueous solution.

Unless specified in the examples, the reaction temperature is room temperature, which is 20°C to 30°C.

The progress of reactions in the examples is monitored by thin layer chromatography (TLC). The systems of the developing agents used for the reactions are: A: dichloromethane and methanol system, B: *n*-hexane and ethyl acetate system, C: petroleum ether and ethyl acetate system, D: acetone. The volume ratio of the solvents is adjusted according to the polarity of the compound.

The eluent system of column chromatography and the developing solvent system of thin layer chromatography used to purify the compounds include: A: dichloromethane and methanol system, B: petroleum ether, ethyl acetate and dichloromethane system, C: petroleum ether and ethyl acetate system. The volume ratio of the solvents is adjusted according to the polarity of the compound. A small amount of triethylamine, acetic acid or other basic or acidic reagents can also be added for adjustment.

EXAMPLES

Example 1: Preparation of ((1*S*,2*R*)-2-fluorocyclopropyl)(3-(2-((1-methyl-1*H*-pyrazol-4-yl)amino)pyrimidin-4-yl)-8-azabicyclo[3.2.1]oct-2-en-8-yl)methanone (**1**)

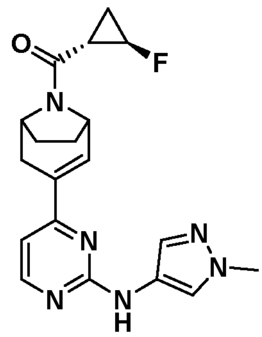

1

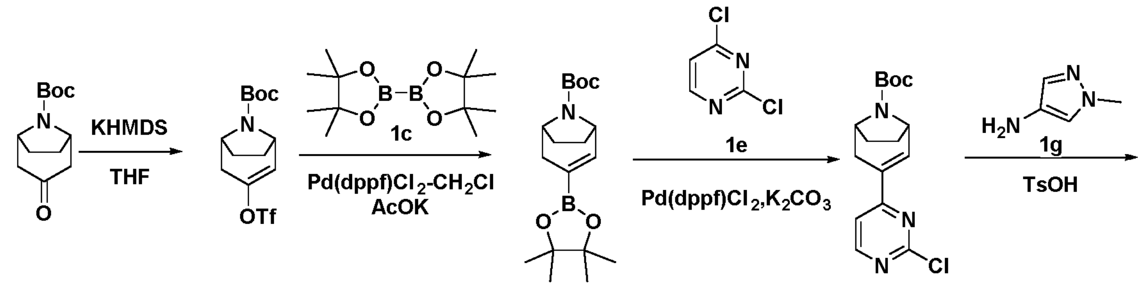


1a 1b 1d 1f

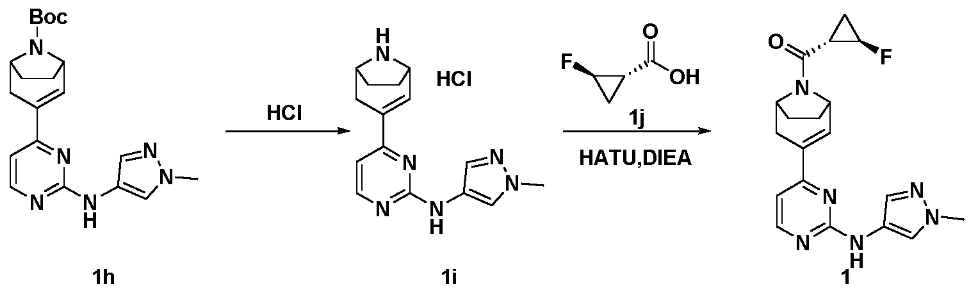


1h 1i 1

Step 1: Synthesis of *tert*-butyl 3-(((trifluoromethyl)sulfonyl)oxy)-8-azabicyclo[3.2.1]oct-2-ene-8-carboxylate (**1b**)

Potassium hexamethyldisilazide (KHMDS, 10.7 mL, 10.7 mmol) was added to a mixed solution of *tert*-butyl 3-oxo-8-azabicyclo[3.2.1]octane-8-carboxylate (2.00 g, 8.88 mmol) in anhydrous tetrahydrofuran (30 mL) at -78°C under nitrogen atmosphere, and the mixture was stirred at -78°C for 0.5 hour. A solution of *N*-phenylbis (trifluoromethanesulfonyl)imide (3.82 g, 10.7 mmol) in anhydrous tetrahydrofuran (20 mL) was added dropwise, and after completion of the addition, the mixture was stirred at -78°C for 2 hours. The reaction was quenched by adding a saturated aqueous solution of ammonium chloride (20 mL). The mixture was extracted with ethyl acetate (30 mL×3). The organic phase was washed with a solution of potassium hydroxide (1 mol/L) and saturated brine, dried over anhydrous sodium sulfate, filtered and concentrated under reduced pressure. The residues were purified by flash column chromatography (mobile phase: petroleum ether/ethyl acetate, 10/1 to 2/1) to obtain 3.10 g of the title compound as a pale yellow oil. Yield: 97.8%.

Step 2: Synthesis of *tert*-butyl 3-(4,4,5,5-tetramethyl-1,3,2-dioxaborolan-2-yl)-8-azabicyclo[3.2.1.]oct-2-ene-8-carboxy late (**1d**)

Pd(dppf)$Cl_2$dichloromethane complex (423 mg, 0.518 mmol) was added to a solution of compound **1b** (3.70 g, 10.4 mmol), potassium acetate (3.05 g, 31.1 mmol) and pinacol diborate (**1c**) (2.90 g, 11.4 mmol) in dioxane (50 mL) under nitrogen atmosphere at room temperature, and the mixture was stirred overnight at 80°C. The solvent was removed by rotary evaporation. The mixture was added to water (40 mL) and extracted with ethyl acetate (50 mL×3). The organic phase was washed with saturated brine, dried over anhydrous sodium sulfate, filtered and concentrated under reduced pressure. The residues were purified by flash column chromatography (mobile phase: petroleum ether/ethyl acetate=10/1 to 2/1) to obtain 1.20 g of the title compound as a yellow oil. Yield: 34.6%.

Step 3: Synthesis of *tert*-butyl 3-(2-chloropyrimidin-4-yl)-8-azabicyclo[3.2.1]oct-2-ene-8-carboxylate (**1f**)

Pd(dppf)$Cl_2$ (262 mg, 0.358 mmol) was added to a mixed solution of compound **1d** (1.20 g, 3.58 mmol), potassium carbonate (1.24 g, 8.95 mmol) and 2,4-dichloropyrimidine (**1e**) (534 mg, 3.58 mmol) in dioxane (40 mL) and water (10 mL) under nitrogen atmosphere at room temperature, and the mixture was stirred overnight at 80°C. The solvent was removed by rotary evaporation. The mixture was added to water (40 mL) and extracted with ethyl acetate (50 mL×3). The organic phase was washed with saturated brine, dried over anhydrous sodium sulfate, filtered and concentrated under reduced pressure. The residues were purified by flash column chromatography (mobile phase: petroleum ether/ethyl acetate=10/1 to 1/1) to obtain 830 mg of the title compound as a yellow oil. Yield: 72.1%.

Step 4: Synthesis of *tert*-butyl 3-(2-((1-methyl-1*H*-pyrazol-4-yl)amino)pyrimidin-4-yl)-8-azabicyclo[3.2.1]oct-2-ene-8-carboxylate (**1h**)

*p*-Toluenesulfonic acid (TsOH, 37.3 mg, 0.217 mmol) was added to a solution of compound **1f** (700 mg, 2.17 mmol) and 1-methyl-1H-pyrazol-4-amine (1**g**) (211 mg, 2.17 mmol) in dioxane (10 mL) at room temperature, and the mixture was stirred at 90°C overnight. The solvent was removed by rotary evaporation. The mixture was added to water (40 mL) and extracted with ethyl acetate (50 mL×3). The organic phase was washed with saturated brine, dried over anhydrous sodium sulfate and concentrated under reduced pressure. The residues were purified by flash column chromatography (mobile phase: petroleum ether/ethyl acetate=10/1 to 1/1) to obtain 600 mg of the title compound as a brown oil. Yield: 72.4%.

Step 5: Synthesis of 4-(8-azabicyclo[3.2.1]oct-2-en-3-yl)-*N*-(1-methyl-1*H*-pyrazol-4-yl)pyrimidin-2-amine hydrochloride **(1i)**

HCl/1,4-dioxane (6 mL, 4 M) was added to a solution of compound **1h** (200 mg, 0.524 mmol) in dichloromethane (6 mL) at room temperature. The mixture was stirred for 30 minutes and concentrated at low temperature to obtain 157 mg of the crude title compound as a white solid.

Step 6: Synthesis of ((1*S*,2*R*)-2-fluorocyclopropyl)(3-(2-((1-methyl-*1*H-pyrazol-4-yl)amino)pyrimidin-4-yl)-8-azabicyclo[3.2.1]oct-2-en-8-yl)methanone (**1**)

2-(7-Oxidobenzotriazole)-*N,N,N',N'*-tetramethylurea hexafluorophosphate (HATU, 224 mg, 0.590 mmol) was added to a solution of (1*S*,2*R*)-2-fluorocyclopropane-1-carboxylic acid (**1j**) (51 mg, 0.492 mmol) in *N,N*-dimethylformamide (10 mL) at room temperature, and the mixture was stirred at room temperature for 30 minutes. Compound **1i** (132 mg, 0.414 mmol) and *N,N*-diisopropylethylamine (DIEA, 190 mg, 1.48 mmol) were added, and the mixture was stirred at room temperature overnight. The mixture was added to water (50 mL) and extracted with ethyl acetate (30 mL×3). The organic phase was washed with saturated brine, dried over anhydrous sodium sulfate, filtered and concentrated under reduced pressure. The residues were purified by preparative liquid chromatography (column: 30mm×250mm; packing: C18, 10 μm; method: 2-22 min, acetonitrile 10-40%; wavelength: 254 nm; flow rate: 45 mL/min; mobile phase: acetonitrile, water) to obtain 95.0 mg of the title compound as a yellow solid. Yield: 59.4%.

LC-MS: m/z 369 $[M+H]^+$;
$^1$H NMR (300 MHz, DMSO-$d_6$): δ ppm 9.34 (s, 1H), 8.34-8.32 (m ,1H), 7.81 (s, 1H), 7.49 (s, 1H), 7.28-7.11(m, 1H), 6.84-6.78 (m, 1H), 5.94-4.61 (m, 3H), 3.45 (s, 3H), 2.92-2.83 (m, 1H), 2.63-2.49 (m, 2H), 2.39-2.25 (m, 1H) , 2.18-2.04 (m, 1H), 2.01-1.83 (m, 1H), 1.75-1.63 (m, 1H), 1.37-1.19 (m, 1H), 1.11-1.07 (m, 1H).

Examples 1-a and 1-b: Preparation of ((1*S*,2*R*)-2-fluorocyclopropyl)((1*S*,5*R*)-3-(2-((1-methyl-1*H*-pyrazol-4-yl)amino)pyrimid in-4-yl)-8-azabicyclo[3.2.1]oct-2-en-8-yl)methanone and ((1*S*,2*R*)-2-fluorocyclopropyl)((1*R*,5*S*)-3-(2-((1-methyl-1*H*-pyrazol-4-yl)amino)pyrimid in-4-yl)-8-azabicyclo[3.2.1]oct-2-en-8-yl)methanone

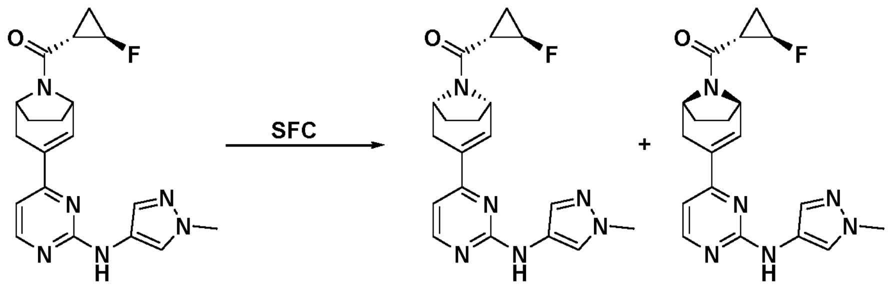


1 1-a and 1-b

Compounds 1-a and 1-b were resolved from compound 1 by supercritical fluid chromatography (SFC).

SFC resolution conditions: column model: AD-H 4.6 mm × 250 mm, 5 μm, mobile phase: MeOH (0.2% $NH_3.H_2O$)/$CO_2$ = 40:60, flow rate: 40 g/min, column temperature: 40°C.

Compound **1-a:**

Retention time: 14.97 min.

LC-MS: m/z 369[M+H]$^+$.

$^1$H NMR (300 MHz, DMSO-*d*$_6$): 9.32 (s, 1H), 8.31 (d, *J* = 5.20 ,1H), 7.79 (d, *J* = 5.60 ,1H), 7.47 (s, 1H), 7.17-7.14(m, 1H), 6.80-6.77 (m, 1H), 5.00-4.61 (m, 3H), 3.77 (s, 3H), 2.90-2.80(m, 1H),2.62-2.47 (m, 2H), 2.36-2.30 (m, 1H) , 2.22-2.05(m, 1H), 2.00-1.86(m, 1H) , 1.76-1.62(m, 1H) , 1.35-1.17(m, 1H) , 1.07-1.04(m, 1H).

Compound **1-b:**

Retention time: 17.98 min.

LC-MS: m/z 369[M+H]$^+$.

$^1$H NMR (300 MHz, DMSO-*d*$_6$): 9.36 (s, 1H), 8.35-8.33 (m ,1H), 7.82 (s,1H), 7.51 (s, 1H), 7.30-7.05(m, 1H), 6.85-6.81 (m, 1H), 4.90-4.63 (m, 3H), 3.09 (s, 3H), 2.95-2.85(m, 1H), 2.65-2.58 (m, 2H), 2.40-2.20 (m, 1H), 2.10-2.00 (m, 1H) , 2.00-1.86 (m, 1H) , 1.76-1.62 (m, 1H) , 1.42-1.33 (m, 1H) , 1.19-1.07 (m, 1H).

Example 2: Preparation of ((1*R*,2*S*)-2-fluorocyclopropyl)(3-(2-((1-methyl-1*H*-pyrazol-4-yl)amino)pyrimidin-4-yl)-8-azabicyclo[3.2.1]oct-2-en-8-yl)methanone (**2**)

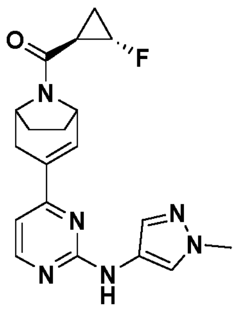

**2**

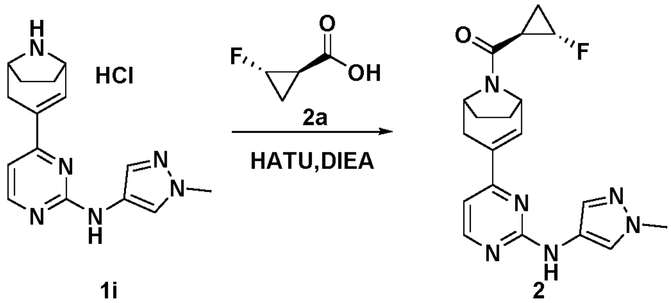


**1i** **2**

The same preparation method as that in Example 1 was used to obtain the title compound **2,** except that (1*S*,2*R*)-2-fluorocyclopropane-1-carboxylic acid (**1j**) was replaced with (1*R*,2*S*)-2-fluorocyclopropane-l-carboxylic acid (**2a**).

Preparative liquid chromatography method: column: 30mm×250mm; packing: C18, 10 μm; method: 2-22 min, acetonitrile 10-50%; wavelength: 254 nm; flow rate: 45 mL/min; mobile phase: acetonitrile, water.

LC-MS: m/z 365 $[M+H]^+$.

$^1H$ NMR (300 MHz, DMSO-$d_6$): δ ppm 9.34 (s, 1H), 8.34-8.30 (m, 1H), 7.83 (s, 1H), 7.51 (s, 1H), 7.18-7.12 (m, 1H), 6.84-6.80 (m, 1H), 4.98-4.64 (m, 3H), 3.80 (s, 3H), 2.93-2.87 (m, 1H), 2.68-2.50 (m, 2H), 2.35-2.24 (m, 1H), 2.08-2.05 (m, 1H), 1.92-1.84 (m, 1H), 1.80-1.61 (m, 1H), 1.41-1.34 (m, 1H), 1.18-1.05 (m, 1H).

Examples 2-a and 2-b: Preparation of ((1*R*,2*S*)-2-fluorocyclopropyl)((1*S*,5R)-(3-(2-((1-methyl-1*H*-pyrazol-4-yl)amino)pyrimid in-4-yl)-8-azabicyclo[3.2.1]oct-2-en-8-yl)methanone and ((1*R*,2*S*)-2-fluorocyclopropyl)((1*R*,5*S*)-3-(2-((1-methyl-1*H*-pyrazol-4-yl)amino)pyrimid in-4-yl)-8-azabicyclo[3.2.1]oct-2-en-8-yl)methanone

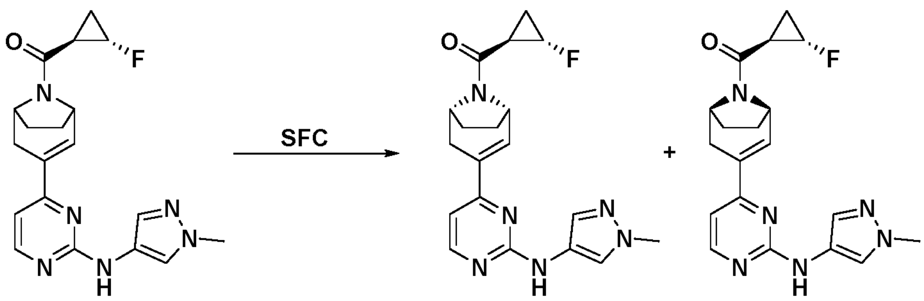


**2** **2-a and 2-b**

Compounds **2-a** and **2-b** were resolved from compound **2** by supercritical fluid chromatography (SFC).

SFC resolution conditions: column model: AD-H 4.6 mm × 250 mm, 5 μm, mobile phase: MeOH (0.2% $NH_3.H_2O$)/$CO_2$ = 40:60, flow rate: 40 g/min, column temperature: 40°C.

Compound **2-a:**

Retention time: 12.53 min.

LC-MS: m/z 369 $[M+H]^+$.

$^1H$ NMR (400 MHz, DMSO): δ9.35 (s, 1H), 8.35-8.32 (m, 1H), 7.82 (s, 1H), 7.50 (s, 1H), 7.19-7.15 (m, 1H), 6.85-6.81 (m, 1H), 4.90-4.63 (m, 3H), 3.81 (s, 3H), 2.91-2.89 (m, 1H), 2.67-2.51 (m, 2H), 2.47-2.29 (m, 1H), 2.09-2.04 (m, 1H), 1.91-1.81 (m, 1H), 1.74-1.58 (m, 1H), 1.45-1.31 (m, 1H), 1.14-1.08 (m, 1H).

Compound **2-b:**

Retention time: 15.46 min.

LC-MS: m/z 369 $[M+H]^+$.

$^1H$ NMR (400 MHz, DMSO): δ 9.35 (s, 1H), 8.35-8.33 (m, 1H), 7.82 (s, 1H), 7.50 (s, 1H), 7.19-7.15 (m, 1H), 6.83-6.81 (m, 1H), 5.03-4.65 (m, 3H), 3.80 (s, 3H), 2.91-2.89 (m, 1H), 2.67-2.51 (m, 2H), 2.48-2.28 (m, 1H), 2.10-2.01 (m, 1H), 1.98-1.86 (m, 1H), 1.84-1.65 (m, 1H), 1.38-1.33 (m, 1H), 1.20-1.06 (m, 1H).

## BIOLOGICAL EVALUATION

Test Example 1: Determination of *in vitro* inhibitory activity of the compounds of the present invention on JAK1 kinase

Experimental materials: JAK1 kinase (Invitrogen, PV4744), ATP (Promega, V915B), ADP-Glo Kinase Assay (Promega, V9101), IRS1 (Signalchem, 140-58-1000).

Sample preparation: The compounds of the present invention and the control product were dissolved in DMSO solvent respectively to formulate into 10 mM mother liquor. The final reaction maximum concentration of the compound was 10 μM, 3-fold dilution, 10 concentration gradients, and duplicate wells for each concentration gradient.

Experimental process: 0.1 μL of the compound to be tested was transferred into a 384-well reaction plate (PE, 6007290) via Echo and centrifuged at 1000 rpm/min for 1 min. 5 μL of JAK1 kinase (final concentration of 4 nM) was transferred into the 384-well reaction plate, which was then centrifuged at 1000 rpm/min for 1 min, and incubated at 25°C for 15 min. 5 μL of substrate mixture (1 mM ATP, IRS1 0.05 mg/ml, kinase buffer solution) was transferred into the 384-well reaction plate, which was then centrifuged at 1000 rpm/min for 1 min, and incubated at 25°C for 60 min. 10 μL of ADP-Glo was transferred into the 384-well reaction plate, which was then centrifuged at 1000 rpm/min for 1 min, and incubated at 25°C for 40 min. 20 μL of test solution was transferred into the 384-well reaction plate, which was then centrifuged at 1000 rpm/min for 1 min and incubated at 25°C for 40 min. RLU (Relative luminescence unit) signal was read by using an Envision multifunctional plate reader. The signal intensity was used to characterize the degree of the kinase activity.

The $IC_{50}$ (half inhibitory concentration) of the compounds was obtained by using the following non-linear fitting equation:

$$Y=Bottom + (Top-Bottom)/(1+10^{\wedge}((LogIC_{50}-X)*HillSlope));$$

X: Log value of the concentration of the compound;
Y: Emission ratio;
Bottom: The minimum value, Top: The maximum value, HillSlope: Slope;

The inhibitory activity of the compounds of the present invention on JAK1 kinase is as shown in Table 1 below. $IC_{50}$ value of 0-100 nM is shown as A, $IC_{50}$ value of 100-300 nM is shown as B, $IC_{50}$ value of 300-1000 nM is shown as C, and $IC_{50}$ value which is greater than 1000 nM is shown as D. NT means not tested.

Table 1: Inhibitory activity of the compounds of the present invention on JAK1 kinase

| Compound No. | $IC_{50}$ (nM) |
|---|---|
| | JAK1 |
| 1 | A |
| 1-a | A |
| 1-b | A |
| 2 | C |

(continued)

| Compound No. | $IC_{50}$ (nM) |
|---|---|
| | JAK1 |
| 2-a | C |
| 2-b | B |

It can be seen from the above experimental results that the compounds of the present invention have good *in vitro* anti-JAK1 kinase activity.

Test Example 2: Inhibition effect of the compounds of the present invention on STAT3 signaling pathway of human whole blood

Experimental materials: CD3 (BD, 555335), pSTAT3 antibody (BD, 612569), IFN-2a (Biolegend, 592702).

Sample preparation: The compounds of the present invention and the control were dissolved in DMSO solvent respectively to formulate into 10 mM mother liquor. The final reaction maximum concentration of the compound was 10 μM, 3-fold dilution, 10 concentration gradients, and duplicate wells for each concentration gradient.

Experimental process: 20 μL of the compound of the present invention was added to a flow cytometer tube (BD, 352052) containing 180 μL of anticoagulant sodium heparin. 20 μL of PBS was added to the control tube. The sample was incubated at 37°C for 30 min. 2 μL of stimulating factor was added and then incubated at 37°C for 20 min. 1 mL of erythrocyte lysate was added, and the sample was repeatedly inverted 5 to 10 times or vortexed and then incubated at 37°C for 10 min. The sample was centrifuged at 600 g for 6 to 8 min. The supernatant was discarded. The mixture was vortexed until the precipitate was suspended. The cells were washed with 3 mL of PBS and centrifuged at 600 g for 6 to 8 min. The supernatant was discarded. The mixture was vortexed until the precipitate was suspended. 1 mL of membrane breaking solution was added, and the system was mixed gently, incubated on ice for 30 min, and centrifuged at 600 g for 6 to 8 min. The supernatant was discarded. The mixture was vortexed until the precipitate was suspended. The cells were washed. 3 mL of PBS was added. The system was centrifuged at 600 g for 6 to 8 min. The supernatant was discarded. The mixture was vortexed until the precipitate was suspended. The process was repeated twice. 100 μL of PBS was added to each staining tube. IFN-2a was added for stimulation, and CD3 and pSTAT3 antibodies (20 μL) were added. The system was mixed well, protected from light and incubated at room temperature for 60 min. The cells were washed. 3 mL of PBS was added. The system was centrifuged at 600 g for 6 to 8 min. The supernatant was discarded. The mixture was vortexed until the precipitate was suspended. The precipitate was suspended in 150 μL in the dark for the flow cytometry analysis. The $IC_{50}$ (half inhibitory concentration) of the compounds was obtained by using the following non-linear fitting equation:

$$Y=Bottom + (Top-Bottom)/(1+10^{\wedge}((LogIC_{50}-X)*HillSlope));$$

X: Log value of the concentration of the compound;
Y: Emission ratio;
Bottom: The minimum value, Top: The maximum value, HillSlope: Slope.

The inhibition effect of the compounds of the present invention on the IFN-2α-stimulated TYK2/JAK1-mediated pathway is shown in Table 2 below. $IC_{50}$ value of 0-100 nM is shown as A, $IC_{50}$ value of 100-300 nM is shown as B, $IC_{50}$ value of 300-1000 nM is shown as C, $IC_{50}$ value which is greater than 1000 nM is shown as D. NT means not tested.

Table 2: Inhibition effect of the compounds of the present invention on STAT3 signaling pathway of human whole blood

| Cytokine/pSTAT | JAK signal path | $IC_{50}$ (nM) | |
|---|---|---|---|
| | | Example 1-a | Example 1-b |
| IFN-2α induced pSTAT3 | TYK2/JAK 1 | A | A |

As can be seen from Table 2, the compounds of the present invention have very good inhibitory effect on the TYK2/JAK1-mediated signaling pathway of human whole blood.

Test Example 3: Pharmacokinetic study in rats

Sample preparation: The compounds of Examples 1-a and 1-b were formulated into 0.2 mg/mL and 0.5 mg/mL solutions with 20% cyclodextrin.

Experimental method: Male SD rats (Beijing Vital River Laboratory Animal Technology Co., Ltd.) were used, the intravenous dose was 1 mg/kg (intravenously, i.v). Blood was collected from the canthus venous plexus before administration and at 0, 5 minutes, 15 minutes, 30 minutes, 1 hour, 2 hours, 4 hours, 6 hours and 8 hours after administration. The oral dose was 5 mg/kg (oral, p.o). Blood was collected from the canthus venous plexus before administration and at 15 minutes, 30 minutes, 1 hour, 2 hours, 4 hours, 6 hours and 8 hours after administration. The blood was anticoagulated with sodium heparin, centrifuged at 3500 rpm for 10 minutes at 4°C. The plasma was obtained and stored at -20°C until testing.

50 μL of the plasma sample melted at room temperature was precisely measured and placed in a 1.5 mL EP tube, followed by adding 400 μL of verapamil internal standard working solution (China National Institute for Drug Control). The EP tube was vortexed vigorously for 1 minute, and centrifuged at 16000 rpm for 10 minutes. The supernatant was collected, filtered with a 0.22 μm organic membrane (AS081320-T, Agela Technologies), and added to an injection vial. The plasma concentration was obtained by LC/MS (Waters UPLC I Class/ TQ-S micro). The main pharmacokinetic parameters of compounds 1-a and 1-b in rats were obtained by DAS3.3.0 software. See Table 3 below.

Table 3 shows the pharmacokinetic parameters of compounds 1-a and 1-b in rats, wherein AUC<500 μg/L*h is shown as C, AUC between 500-1000 μg/L*h is shown as B, AUC>1000 μg/L*h is shown as A; $C_{max}$<300 μg/L is shown as C, $C_{max}$ between 300-500 μg/L is shown as B, and $C_{max}$>500 μg/L is shown as A; $T_{1/2}$ <1 h is shown as B, and $T_{1/2}$ >1 h is shown as A; F <30% is shown as C, F between 30-50% is shown as B, and F >50% is shown as A.

Table 3: Pharmacokinetic parameters of compounds 1-a and 1-b of the present invention in rats

| Examples | 1-a | 1-b |
|---|---|---|
| $AUC_{0-t}$, μg/L*h | A | B |
| $C_{max}$, μg/L | A | B |
| $T_{1/2}$(h) | A | A |
| F(%) | A | A |

It can be seen from Table 3 that compounds 1-a and 1-b of the present invention have good pharmacokinetic properties, and are suitable for oral administration.

Test Example 4: AIA pharmacodynamic studies of the compounds of the present invention in rats

Experimental materials: Complete Freund's adjuvant (Chondrex, 7027); positive control compound PF-06700841, which has a structure of

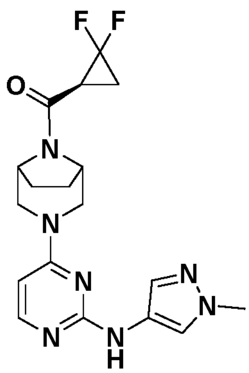

,

and is prepared according to J Med Chem, 2018, 61 8597-8612.

Experimental process: The *in vivo* efficacy of the compounds of the present invention was investigated. 8-Week-old female Lewis rats (Beijing Vital River Laboratory Animal Technology Co., Ltd.) were subcutaneously injected with 0.1 mL of complete Freund's adjuvant at a single site on the base of their tails, and grouped 13 days after modeling. 8 groups were divided: the normal group, the model group, the PF-06700841 low dose group, the PF-06700841 high

dose group, the compound 1-a low dose group, the compound 1-a high dose group, the compound 1-b low dose group, and the compound 1-b high dose group (the compounds to be tested were formulated into 0.1 mg/mL and 0.3 mg/mL solutions with 20% cyclodextrin solution). 20% cyclodextrin was administered to the model group, 1 mg/kg of the test compound was administered to the low dose group, and 3 mg/kg of the test compound was administered to the high dose group. The scores were evaluated every 2 days after administration. Scoring criteria: 0 point: no redness and swelling; 1 point: mild redness and swelling of ankle joints and wrist joints; 2 points: moderate redness and swelling of ankle joints and wrist joints; 3 points: severe redness and swelling of the paw including the fingertips; 4 points: maximal inflammation of the extremities, including multiple joints. The scoring results were analyzed by Graph prism9.0 software.

Figure 1 is a graph showing the rat AIA pharmacodynamic scoring results of the compounds of the present invention. It can be seen from the results of the *in vivo* efficacy experiment in the AIA model that in the low dose groups and high dose groups, the efficacy of the compounds 1-a and 1-b of the present invention is significantly better than that of the reference PF-06700841. At the same time, the efficacy of isomer 1-a is significantly better than that of isomer 1-b.

## Claims

1. A compound or a pharmaceutically acceptable salt thereof selected from the group consisting of:

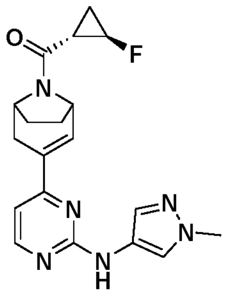 ,

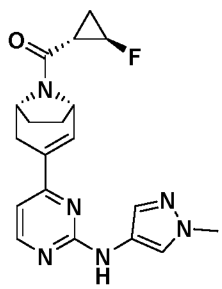 ,

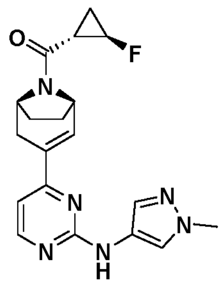 ,

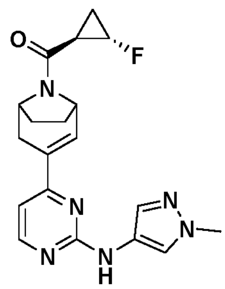 ,

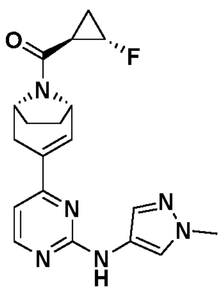 and

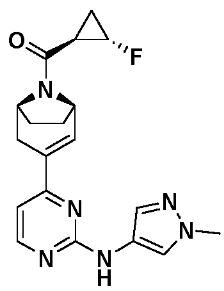 .

2. A method for preparing the compound of formula 1 or a pharmaceutically acceptable salt thereof, comprising the following step of:

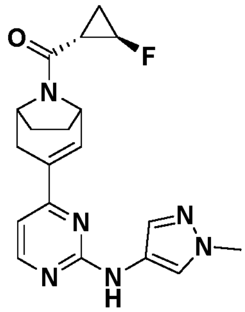

1

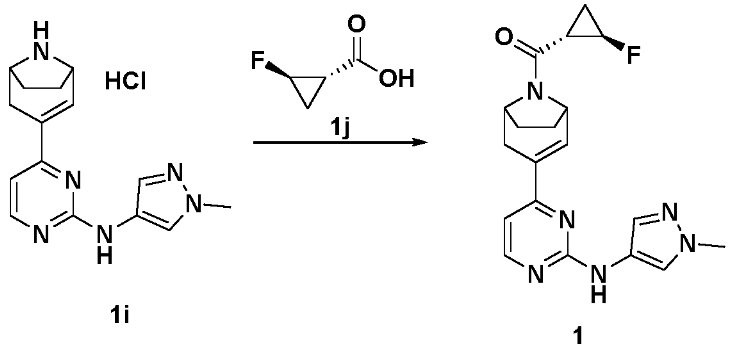


1i

1

reacting the hydrochloride of compound **1i** with compound 1j under alkaline conditions and the presence of a catalyst to obtain compound 1; the reagent providing alkaline conditions is preferably DIEA, the catalyst is preferably HATU.

**3.** A method for preparing the compound of formula 2 or a pharmaceutically acceptable salt thereof, comprising the following step of:

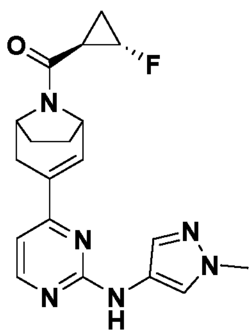

2

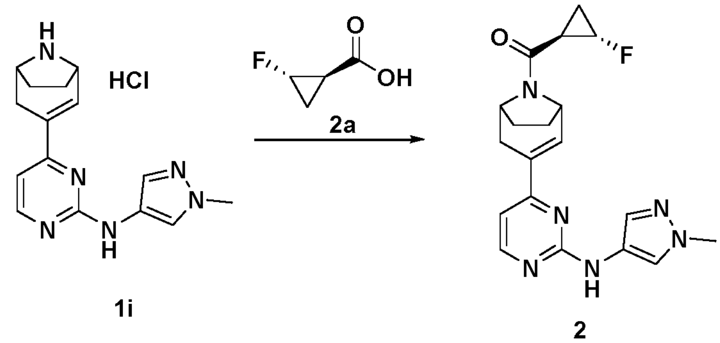


1i

2

reacting the hydrochloride of compound **1i** with compound **2** under alkaline conditions and the presence of a catalyst to obtain compound **2;** the reagent providing alkaline conditions is preferably DIEA, the catalyst is preferably HATU.

4. A method for preparing the compound of formula 1-a and/or 1-b or a pharmaceutically acceptable salt thereof, comprising the following step of:

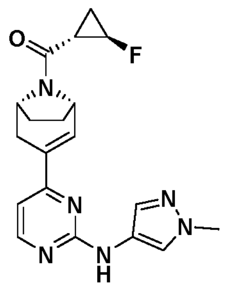

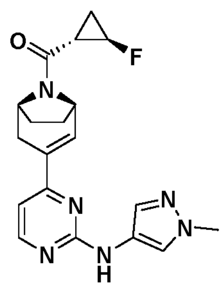

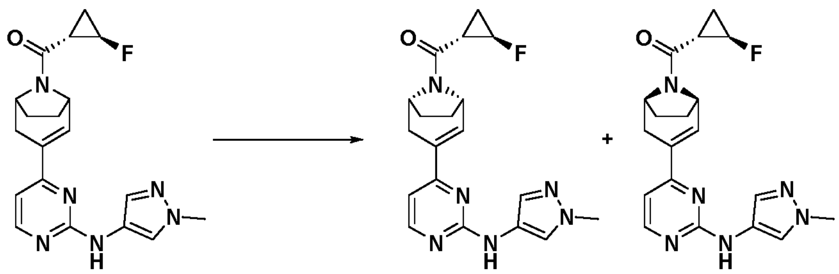

1

resolving compound **1** by supercritical fluid chromatography to obtain compound 1-a and/or compound 1-b; wherein the one with shorter retention time is compound 1-a, and the one with longer retention time is compound 1-b.

5. A method for preparing the compound of formula 2-a and/or 2-b or a pharmaceutically acceptable salt thereof, comprising the following step of:

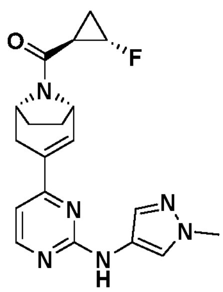

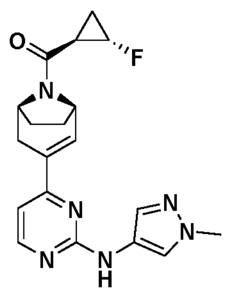

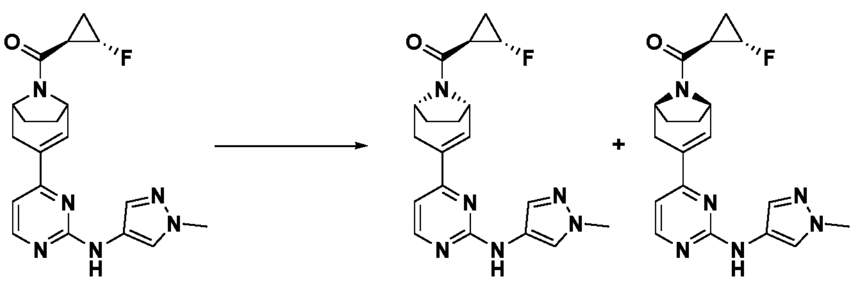

2

resolving compound **2** by supercritical fluid chromatography to obtain compound 2-a and/or compound 2-b; wherein the one with shorter retention time is compound 2-a, and the one with longer retention time is compound 2-b.

6. The method according to claim 4 or 5, wherein the mobile phase in supercritical fluid chromatography is methanol and carbon dioxide, and preferably methanol containing ammonia and carbon dioxide.

7. A pharmaceutical composition comprising the compound according to claim 1 or a pharmaceutically acceptable salt

thereof and a pharmaceutically acceptable carrier.

**8.** Use of the compound according to claim 1 or a pharmaceutically acceptable salt thereof or the pharmaceutical composition according to claim 7 in the preparation of medicaments for the prevention and/or treatment of diseases related to JAK1 and TYK2 activity.

**9.** The use according to claim 8, wherein the disease is selected from the group consisting of inflammation, autoimmune disease and cancer, and the inflammation is preferably selected from the group consisting of rheumatoid arthritis, psoriatic arthritis, inflammatory bowel disease, uveitis, psoriasis and atopic dermatitis, the autoimmune disease is preferably selected from the group consisting of multiple sclerosis and lupus; the cancer is preferably selected from the group consisting of breast cancer, cervical cancer, colon cancer, lung cancer, gastric cancer, rectal cancer, pancreatic cancer, brain cancer, skin cancer, oral cancer, prostate cancer, bone cancer, kidney cancer, ovarian cancer, bladder cancer, liver cancer, fallopian tube tumor, ovarian tumor, peritoneal tumor, melanoma, solid tumor, glioma, glioblastoma, hepatocellular carcinoma, mastoid nephroma, head and neck tumors, leukemia, lymphoma, myeloma and non-small cell lung cancer.

Figure 1

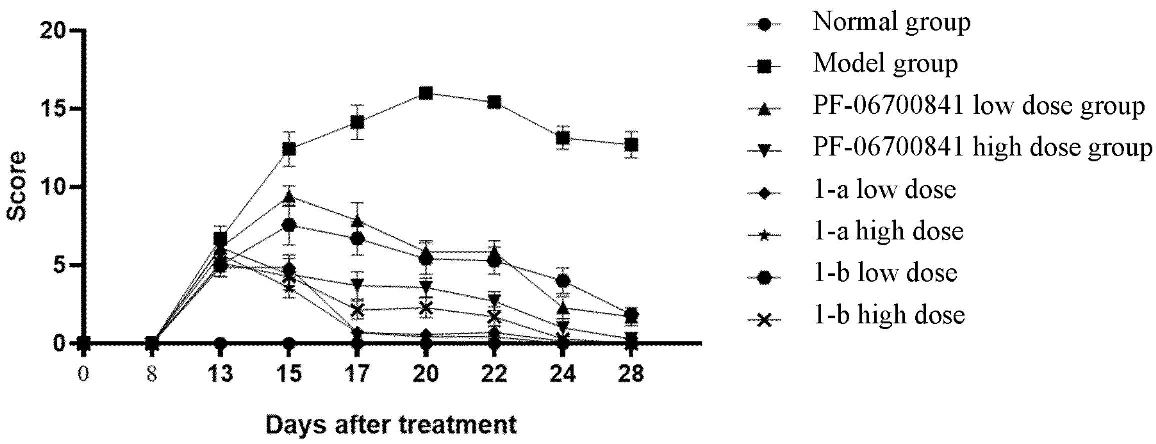
20
15
10
5
0
Score
0
8
13
15
17
20
22
24
28
Days after treatment
Normal group
Model group
PF-06700841 low dose group
PF-06700841 high dose group
1-a low dose
1-a high dose
1-b low dose
1-b high dose

## INTERNATIONAL SEARCH REPORT

International application No.
**PCT/CN2022/071491**

**A. CLASSIFICATION OF SUBJECT MATTER**

C07D 239/42(2006.01)i； C07D 403/12(2006.01)i； A61K 31/506(2006.01)i； A61P 37/00(2006.01)i

According to International Patent Classification (IPC) or to both national classification and IPC

**B. FIELDS SEARCHED**

Minimum documentation searched (classification system followed by classification symbols)

C07D; A61K; A61P

Documentation searched other than minimum documentation to the extent that such documents are included in the fields searched

Electronic data base consulted during the international search (name of data base and, where practicable, search terms used)

WPI, EPODOC, CNABS, CNTXT, CNKI, GOOGLE, REGISTRY, CAPLUS: 甲酮, 氟环丙基, 氮杂双环[3.2.1]辛, 桥杂环+, JAK, brideged heterocyclyl, diazabicyclo[3.2.1]octan, methanone, structure search, 中国医药研究开发中心有限公司, Yin Huijun, Shi Jizhou, Liu Guobiao, Fei Teng, Dong liuxin

**C. DOCUMENTS CONSIDERED TO BE RELEVANT**

| Category* | Citation of document, with indication, where appropriate, of the relevant passages | Relevant to claim No. |
|---|---|---|
| PX | WO 2021027647 A1 (CHINA MEDICINE RESEARCH & DEVELOPMENT CENTER CO., LTD.) 18 February 2021 (2021-02-18)<br>description, page 2, paragraph 4 to page 3, paragraph 2 from the bottom, description, pages 5-32, tables, test examples 1-3 | 1-9 |
| A | CN 107074867 A (PFIZER INC.) 18 August 2017 (2017-08-18)<br>description paragraphs [0058]-[0066], [0341], [0511]-[0517], [1193]-[1219], paragraph [1194] embodiment 7 | 1-9 |
| A | CN 110862376 A (JIAXING TEKELUO BIOTECHNOLOGY CO., LTD.) 06 March 2020 (2020-03-06)<br>paragraphs [0006]-[0091] | 1-9 |
| A | FENSOME Andrew et al. "Dual Inhibition of TYK2 and JAK1 for the Treatment of Autoimmune Diseases: Discovery of ((S)-2, 2-Difluorocyclopropyl)((1R, 5S)-3-(2-((1-methyl-1H-pyrazol-4-yl)amino)pyrimidin-4-yl)-3, 8-diazabicyclo[3.2.1]octan-8-yl)methanone (PF-06700841)"<br>*J. Med. Chem.,* Vol. 61, 16 August 2018 (2018-08-16),<br>pp. 8597-8612 | 1-9 |

☑ Further documents are listed in the continuation of Box C.  ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **29 March 2022** | **13 April 2022** |

| Name and mailing address of the ISA/CN | Authorized officer |
|---|---|
| **China National Intellectual Property Administration (ISA/CN)**<br>**No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

**INTERNATIONAL SEARCH REPORT**

International application No.
**PCT/CN2022/071491**

**C. DOCUMENTS CONSIDERED TO BE RELEVANT**

| Category* | Citation of document, with indication, where appropriate, of the relevant passages | Relevant to claim No. |
|---|---|---|
| PA | CN 113372351 A (MINGHUI PHARMACEUTICAL (HANGZHOU) CO., LTD. et al.) 10 September 2021 (2021-09-10)<br>entire document | 1-9 |

Form PCT/ISA/210 (second sheet) (January 2015)

**INTERNATIONAL SEARCH REPORT**
**Information on patent family members**

International application No.
**PCT/CN2022/071491**

| Patent document cited in search report | Publication date (day/month/year) | Patent family member(s) | Publication date (day/month/year) |
|---|---|---|---|
| WO 2021027647 A1 | 18 February 2021 | TW 202115036 A | 16 April 2021 |
| | | CN 112654605 A | 13 April 2021 |
| CN 107074867 A | 18 August 2017 | NZ 729005 A | 26 June 2020 |
| | | TW 201609706 A | 16 March 2016 |
| | | TN 2017000044 A1 | 04 July 2018 |
| | | US 2016052930 A1 | 25 February 2016 |
| | | HU E035553 T2 | 02 May 2018 |
| | | ES 2655971 T3 | 22 February 2018 |
| | | HR P20171913 T1 | 09 February 2018 |
| | | ZA 201701005 B | 31 July 2019 |
| | | UY 36275 A | 01 April 2016 |
| | | CA 2900855 A1 | 21 February 2016 |
| | | AU 2015304883 A1 | 02 March 2017 |
| | | NO 2721710 T3 | 31 March 2018 |
| | | MA 40587 A | 28 June 2017 |
| | | DO P2017000048 A | 30 June 2017 |
| | | EC SP17010156 A | 30 November 2017 |
| | | AR 101599 A1 | 28 December 2016 |
| | | PH 12017500276 A1 | 03 July 2017 |
| | | PT 3183247 T | 18 January 2018 |
| | | GE P20197003 B | 25 July 2019 |
| | | DK 3183247 T3 | 15 January 2018 |
| | | PL 3183247 T3 | 30 March 2018 |
| | | ME 02856 B | 20 April 2018 |
| | | CL 2017000406 A1 | 06 October 2017 |
| | | SG 11201700957 W A | 30 March 2017 |
| | | WO 2016027195 A1 | 25 February 2016 |
| | | SV 2017005384 A | 10 July 2017 |
| | | KR 20170036109 A | 31 March 2017 |
| | | UA 117976 C2 | 25 October 2018 |
| | | MD 20170016 A2 | 31 August 2017 |
| | | US 2020038409 A1 | 06 February 2020 |
| | | CU 20170015 A7 | 05 June 2017 |
| | | US 2017239264 A1 | 24 August 2017 |
| | | BR 112017003054 A2 | 21 November 2017 |
| | | GT 201700035 A | 10 October 2019 |
| | | AP 201709748 A0 | 28 February 2017 |
| | | CY 1119771 T1 | 27 June 2018 |
| | | US 2020330477 A1 | 22 October 2020 |
| | | JP 2017524022 A | 24 August 2017 |
| | | IL 250709 D0 | 30 April 2017 |
| | | CR 20170066 A | 17 July 2017 |
| | | SI 3183247 T1 | 28 February 2018 |
| | | NI 201700020 A | 02 May 2017 |
| | | MX 2017002241 A | 03 May 2017 |
| | | EA 201700077 A1 | 31 July 2017 |
| | | LT 3183247 T | 25 January 2018 |
| | | RS 56807 B1 | 30 April 2018 |
| | | US 2021377495 A1 | 02 December 2021 |
| | | EP 3183247 A1 | 28 June 2017 |

Form PCT/ISA/210 (patent family annex) (January 2015)

**INTERNATIONAL SEARCH REPORT**
**Information on patent family members**

International application No.
**PCT/CN2022/071491**

| Patent document cited in search report | Publication date (day/month/year) | Patent family member(s) | Publication date (day/month/year) |
|---|---|---|---|
| | | PE 20171177 A1 | 22 August 2017 |
| CN 110862376 A | 06 March 2020 | WO 2021078021 A1 | 29 April 2021 |
| CN 113372351 A | 10 September 2021 | None | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- *J. Immunol.,* 2015, vol. 194, 21
- *J. Med. Chem.,* 2014, vol. 57, 5023
- *Curr. Opin. Rheumatol.,* 2014, vol. 26, 237
- *J Med Chem,* 2018, vol. 61, 8597-8612